(19) Europäisches Patentamt
European Patent Office
Office européen des brevets

(11) Veröffentlichungsnummer : **0 442 431 B1**

(12) **EUROPÄISCHE PATENTSCHRIFT**

(45) Veröffentlichungstag der Patentschrift :
**11.05.94 Patentblatt 94/19**

(51) Int. Cl.$^5$ : **C07C 49/707,** C07C 45/43

(21) Anmeldenummer : **91101903.2**

(22) Anmeldetag : **11.02.91**

# (54) Verfahren zur Herstellung von Quadratsäure.

(30) Priorität : **12.02.90 CH 439/90**

(43) Veröffentlichungstag der Anmeldung :
**21.08.91 Patentblatt 91/34**

(45) Bekanntmachung des Hinweises auf die Patenterteilung :
**11.05.94 Patentblatt 94/19**

(84) Benannte Vertragsstaaten :
**AT BE CH DE DK ES FR GB IT LI LU NL SE**

(56) Entgegenhaltungen :
**EP-A- 0 123 042**
**DE-B- 1 253 266**
**DE-B- 2 623 836**
**DE-B- 2 824 558**
**US-A- 4 104 308**

(73) Patentinhaber : **LONZA AG**
**CH-3945**
**Gampel/Wallis (CH)**

(72) Erfinder : **Scholl, Thomas, Dr.**
**Terbinerstrasse 40**
**Visp, Kanton Wallis (CH)**
Erfinder : **Jackson, Barry, Dr.**
**Jesuitenweg 158**
**Glis, Kanton Wallis (CH)**

(74) Vertreter : **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

**EP 0 442 431 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Quadratsäure, wobei Quadratsäure in hoher Reinheit und mit hoher Ausbeute isoliert wird, sowie halogenierte Cyclobutenone als neue Zwischenprodukte im Verfahren.

Quadratsäure ist ein interessantes Zwischenprodukt zur Herstellung von Pharmazeutika, Farbstoffen (Angew. Chem., 20, 1966, S.931) und Herbiziden (CH-PS 609 837).

Aus der Literatur sind verschiedene Verfahren zur Herstellung von Quadratsäure bekannt.

Mehrere gehen von Hexachlor-1,3-butadien aus, das mit Hilfe von Natriumethanolat zu einem chlorierten Cyclobuten-Derivat zyklisiert wird. Diese Zwischenprodukte werden mit Schwefelsäure oder anderen Säuren zu Quadratsäure hydrolysiert (Roedig, A. und Bernemann, P., Liebigs Ann. Chem. 1956, 600, S.1; Maahs, G., Liebigs Ann. Chem. 1965, 686, S.55; Angew. Chemie, 1963, 75, S.982; Uehara, A. und Tsuchiya, R., Sci. Rep. Kanazawa Univ. 1980, 25, S.83; Fan, R. et al., Chemical Abstracts 1987, 106, 103798c). Statt Natriumethanolat wird auch Morpholin verwendet (Maahs, G. und Hegenberg P., Angew. Chemie 1966, 78, S.927; Schmidt, A.H. und Ried., W., Synthesis 1978, S.869; Gadek, T.R. et al. 1976, US-PS 4 104 308; Paine, A.J., Tetrahedron Letters, 1984, 25 S.135). Die Zyklisierung kann auch rein thermisch erfolgen (Müller, W. 1976,
DE-PS 26 18 557; Schröder, M. und Schäfer, W. 1976,
DE-PS 26 23 836; Maahs, G. und Rombusch, D. 1978,
DE-PS 28 24 558; Rombusch, K. und Maahs, G. 1983
DE-OS 33 14 431).

Nachteile all dieser Verfahren sind entweder bescheidene Ausbeuten oder hoher Aufwand (z.B. Destillation mit extremem Rücklaufverhältnis) und die besonderen Sicherheitsmassnahmen, die beim Umgang mit dem krebserzeugenden Edukt Hexachlor-1,3-butadien erforderlich sind.

Nach einem anderen Verfahren (Belluv, D. et al., Helv. Chim. Acta, 1978, 61, S.1784) wird aus dem Pilzmetaboliten Moniliformin durch Bromierung und Hydrolyse Quadratsäure in 70% Ausbeute gewonnen. Moniliformin kommt aber in der Natur nur in kleinen Mengen vor, und die bekannten Synthesen dafür sind aufwendig und ergeben nur bescheidene Ausbeuten.

Ein weiteres Verfahren, die elektrochemische reduktive Tetramerisierung von Kohlenmonoxid zu Quadratsäure, verlangt einen grossen apparativen Aufwand und ergibt ein Produktegemisch, aus dem Quadratsäure nur schwer in reiner Form isolierbar ist. (Silvestri, G. et al., Gazz. Chim. Itl., 1972, 102, S.818; DE-OS 22 35 882; US-PS 4 461 681, US-PS 4 523 980, Fabre, P.L. et al., Bull. Soc. Chim. Fr., 1988, S.933)

Aufgabe der vorliegenden Erfindung war es, eine neue Synthese von Quadratsäure vorzuschlagen, die von leicht zugänglichen Edukten ausgeht und Quadratsäure in guter Ausbeute und Reinheit ergibt.

Die Aufgabe der Erfindung wurde gelöst, indem man einen Destillationsrückstand der Diketen-Produktion mit einem Gehalt an 3-Acetoxy-2-cyclobuten-1-on, das im folgenden als Triketen bezeichnet wird, der Formel

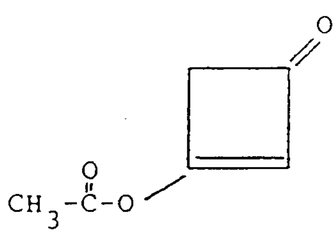

I

oder reines Triketen entweder

a) in einer ersten Stufe durch Halogenierung zu einem Cyclobutenon der allgemeinen Formel

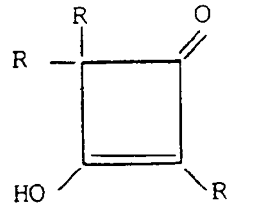

II

worin R gleichbedeutend ist und ein Chlor- oder Bromatom bedeutet, umsetzt, dieses Zwischenprodukt gegebenenfalls isoliert und dann in einer zweiten Stufe zur Quadratsäure hydrolysiert oder

b) in einer ersten Stufe durch saure Hydrolyse in das 1,3-Cyclobutandion überführt, dieses Zwischenprodukt isoliert, dieses dann in einer zweiten Stufe durch Halogenierung zum Cyclobutenon der allgemeinen Formel II umsetzt, dieses Zwischenprodukt gegebenenfalls isoliert und dann in einer dritten Stufe zur Quadratsäure hydrolysiert.

Vorzugsweise wird ein Destillationsrückstand der Diketen-Produktion mit einem Gehalt an Triketen von 5 bis 60 Gew.% als Ausgangsmaterial eingesetzt.

Die Halogenierung von reinem Triketen oder von Triketen aus dem Destillationsrückstand der Diketen-Produktion zum Cyclobutenon gemäss Formel II wird zweckmässig mit 1 bis 4 Molen Brom oder Chlor, bezogen auf 1 Mol Ausgangsmaterial, vorzugsweise mit 2,5 bis 3,5 Molen Brom oder Chlor, insbesondere mit 2,5 bis 3,5 Molen Brom, durchgeführt.
Die Reaktion wird üblicherweise bei einer Temperatur von 10 bis 80°C, vorzugsweise von 15 bis 35°C, durchgeführt.
Nach einer Reaktionszeit von in der Regel 10 bis 180 Minuten, wird entweder das Cyclobutenon gemäss Formel II durch übliches Einengen erhalten, oder der Rückstand wird direkt zur Quadratsäure hydrolysiert.
Für die Halogenierungsreaktionen können als Lösungsmittel $C_1$ - $C_4$-Carbonsäuren, $C_1$ - $C_4$-Carbonsäureester, Carbonsäureanhydride und chlorierte Kohlenwasserstoffe angewendet werden. Als Vertreter dieser Lösungsmittel können beispielsweise Essigsäure, Essigsäureethylester, Acetanhydrid, Chloroform, Methylenchlorid und Tetrachlorkohlenstoff, vorzugsweise Essigsäureethylester oder Essigsäure angewendet werden.
Die saure Hydrolyse von reinem Triketen oder von Triketen aus dem Destillationsrückstand der Diketen-Produktion zum 1,3-Cyclobutandion kann mit wässrigen Säuren wie beispielsweise Ameisensäure oder Schwefelsäure im Ueberschuss durchgeführt werden. Vorzugsweise wird wässrige Ameisensäure im Ueberschuss angewendet. Die Hydrolyse wird üblicherweise bei einer Temperatur von 0 bis 30°C, vorzugsweise von 10 bis 30°C, durchgeführt. Nach einer Reaktionszeit von in der Regel 15 Minuten bis 24 Stunden, wird das 1,3-Cyclobutandion auf übliche Art und Weise aufgearbeitet, z.B. durch Extraktion oder Umkristallisation.
Die zweite Stufe, die Halogenierung von 1,3-Cyclobutandion, wird mit 1 bis 5 Molen Brom oder Chlor, bezogen auf 1 Mol 1,3-Cyclobutandion, vorzugsweise mit 2 bis 4 Molen Brom oder Chlor, insbesondere mit 2,5 bis 3,5 Molen Brom, durchgeführt. Die Halogenierung wird üblicherweise bei einer Temperatur von 0 bis 50°C, vorzugsweise von 0 bis 20°C durchgeführt. Nach einer Reaktionszeit von in der Regel 30 Minuten bis 4 Stunden, wird das Cyclobutenon, gemäss Formel II, in guter Ausbeute durch übliches Einengen erhalten, oder der Rückstand wird direkt zur Quadratsäure hydrolysiert. Als Lösungsmittel können die gleichen wie die zuvor beschriebenen angewendet werden.

Die Cyclobutenone gemäss Formel

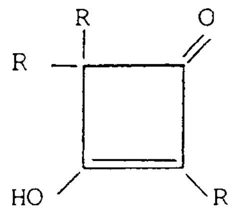

II

worin R gleichbedeutend ist und ein Chlor- oder Bromatom bedeutet, sind neu und lassen sich zur Quadratsäure hydrolysieren, wobei Quadratsäure in guter Ausbeute und Reinheit isoliert werden kann.

Die Cyclobutenone der allgemeinen Formel II sind 2,4,4-Tribrom-3-hydroxy-2-cyclobuten-1-on und 2,4,4-Trichlor-3-hydroxy-2-cyclobuten-1-on.

Die Hydrolyse zur Quadratsäure kann mit Mineralsäuren, wie beispielsweise Schwefelsäure, Salzsäure, Bromwasserstoffsäure oder Phosphorsäure, mit Carbonsäuren, wie beispielsweise mit wässriger Ameisensäure oder wässriger Trifluoressigsäure, mit Sulfonsäuren, wie beispielsweise mit wässriger Methansulfonsäure oder mit Wasser durchgeführt werden. Vorzugsweise werden Mineralsäuren, wie beispielsweise konzentrierte Schwefelsäure oder Salzsäure, im Ueberschuss angewendet.
Die Hydrolyse wird zweckmässig bei einer Temperatur von 50 bis 150°C, vorzugsweise bei einer Temperatur von 80 bis 120°C, durchgeführt.

Die Hydrolyse zur Quadratsäure mit Wasser wird unter Rückfluss durchgeführt. Nach einer Reaktionszeit von 2 bis 48 Stunden wird Quadratsäure in guter Ausbeute erhalten.

Beispiel 1

Anreicherung Triketen

148 g Destillationsrückstand der Diketen-Produktion mit einem Gehalt an Triketen von 15,5 Gew.% wurde auf 10°C gekühlt, und bei einem Druck von 0,1 bis 0,3 mbar wurden pro Stunde 17 ml dieses Rückstandes in den Dünnfilmverdampfer gegeben. Dabei wurden 4 g bräunliche Kristalle mit einem Gehalt von 97% Triketen und 20 g einer gelben Flüssigkeit mit einem Gehalt von 51,5% Triketen abgeschieden. Der Destillationsrückstand wurde bei einer Temperatur von 20 bis 40°C und einem Druck von 0,2 mbar 20 Minuten lang von Diketen und anderen leichtflüchtigen Verunreinigungen befreit, anschliessend in Diethylether (50 ml) gelöst, dann auf -60°C abgekühlt und filtriert. Der Rückstand wurde mit -60°C kaltem Diethylether (10 ml) gewaschen und trockengesaugt. Die 11,7 g bräunlichen Kristalle wurden bei einer Temperatur von 30°C und einem Druck von 0,1 mbar sublimiert. Die Sublimation ergab 10,5 g farblose Kristalle von Triketen, entsprechend einer Ausbeute von 46%, bezogen auf das im Diketen-Harz enthaltene Triketen.
Smp. 34°C

Beispiel 2

Herstellung von 2,4,4-Tribrom-3-hydroxy-2-cyclobuten-1-on

Zu 1,26 g (10 mmol) Triketen, gelöst in Essigsäureethylester (10 ml), wurden innerhalb 20 Minuten unter Rühren 4,84 g Brom (30 mmol), gelöst in Essigsäureethylester (10 ml), hinzugetropft. Die Reaktionstemperatur wurde zwischen 22 und 30 °C gehalten. Nach einer weiteren Stunde Rühren bei Raumtemperatur wurde die orange Lösung am Rotationsverdampfer eingeengt. Nach dem Einengen wurden 3,07 g Produkt, entsprechend einer Ausbeute von 96% bezogen auf eingesetztes Triketen , erhalten.
Schmelzpunkt: 163°C (Zersetzung)
$^{1}$H-NMR ($d_7$-DMF, 300 MHz, $\delta$ in ppm): 11,2, bs
$^{13}$H-NMR($d_7$-DMF, 300 MHz, $\delta$ in ppm): 71, s; 178, s; 209, s; 221, s

Beispiel 3

Herstellung von 1,3-Cyclobutandion

14 g Triketen (Gehalt 95%, 105 mmol) wurden bei 10°C portionenweise zu einem Gemisch von 140 g Ameisensäure und 4,6 g Wasser (256 mmol) gegeben.
Nach 4 Stunden wurde eingeengt. Der braune Rückstand wurde in Diethylether (20 ml) aufgeschlämmt, abfiltriert und mit Diethylether (5 ml) gewaschen.
Nach Trocknung verblieben 8,8 g hellbraune Kristalle mit einem Schmelzpunkt von 118 bis 119°C (Zersetzung) und einem Gehalt von 97% 1,3-Cyclobutandion, was einer Ausbeute von 96%, bezogen auf Triketen, entspricht.

Beispiel 4

Herstellung von 2,4,4-Trichlor-3-hydroxy-2-cyclobuten-1-on

0,84 g (10 mmol) 1,3-Cyclobutandion wurden in Tetrachlorkohlenstoff (10 ml) aufgeschlämmt. Unter Rühren wurde in 45 Minuten bei 2 bis 4°C 2,3 g (32 mmol) Chlor eingeleitet. Nach beendeter Chlorzugabe wurde die gelbe Suspension noch 1 Stunde lang bei 4 bis 9°C gerührt. Durch Einengen am Rotationsdampfer wurden 1,7 g Produkt, entsprechend einer Ausbeute von 90%, bezogen auf eingesetztes 1,3-Cyclobutandion, erhalten.
Schmelzpunkt: 156 bis 159°C (Zersetzung)
$^{1}$H-NMR ($d_7$-DMF, 300 MHz, $\delta$ in ppm): 9,8, s

Beispiel 5

Herstellung von Quadratsäure durch Hydrolyse mit Schwefelsäure

a) ausgehend von 2,4,4-Tribrom-3-hydroxy-2-cyclobuten-1-on

Zu 3,07 g (9,6 mmol) 2,4,4-Tribrom-3-hydroxy-2-cyclobuten-1-on wurde konzentrierte Schwefelsäure (99%, 5 ml) hinzugegeben. Diese Suspension wurde 15 Stunden lang bei 100°C gerührt, dann abgekühlt

und filtriert. Der Rückstand wurde 3 mal mit Aceton (je 1 ml) gewaschen und anschliessend bei 50°C und 40 mbar 4 Stunden lang getrocknet. Es wurden 1,03 g weisse Kristalle mit einem Gehalt von 95% Quadratsäure erhalten, entsprechend 0,97 g (8,5 mmol) Quadratsäure und einer Ausbeute von 85%, bezogen auf eingesetztes Triketen.

b) ausgehend von 2,4,4-Trichlor-3-hydroxy-2-cyclobuten-1-on

Zu 1,7 g (9 mmol) 2,4,4-Trichlor-3-hydroxy-2-cyclobuten-1-on wurde konzentrierte Schwefelsäure (99%, 4 ml) und Wasser (0,5 ml) hinzugegeben. Diese Suspension wurde 15 Stunden lang bei 80°C gerührt, abgekühlt und filtriert. Der Rückstand wurde 3 mal mit Aceton (je 2 ml) gewaschen und anschliessend bei 50°C und 40 mbar 15 Stunden lang getrocknet. Es wurden 0,71 g grauer Festkörper mit einem Quadratsäuregehalt von 93% erhalten, entsprechend 0,66 g (5,8 mmol) reiner Quadratsäure und einer Ausbeute von 58%, bezogen auf 1,3-Cyclobutandion.

Beispiel 6

Herstellung von Quadratsäure durch Hydrolyse mit Wasser

Zu 1,30 g (4,1 mmol) 2,4,4-Tribrom-3-hydroxy-2-cyclobuten-1-on wurden 10 ml Wasser gegeben. Die gelbliche Lösung wurde 14 Stunden unter Rückfluss gekocht, auf 5°C abgekühlt und filtriert. Der Rückstand wurde zweimal mit je 0,5 ml Wasser gewaschen und dann bei 50°C und 40 mbar 16 Stunden lang getrocknet. Es verblieben 0,344 g graue Kristalle, die 96% Quadratsäure enthielten, was 0,330 g (2,9 mmol) Quadratsäure und einer Ausbeute von 70%, bezogen auf eingesetztes Triketen, entspricht.

Beispiel 7

Herstellung von Quadratsäure durch Hydrolyse mit weiteren Säuren

Analog zu Beispiel 5 a wurde Quadratsäure durch Hydrolyse von je 0,50 g (1,6 mmol) 2,4,4-Tribrom-3-hydroxy-2-cyclobuten-1-on hergestellt.

Ergebnisse:

| Wässrige Säure Name | Gehalt | g | Ausbeute an Quadratsäure bez. auf einges. Triketen |
|---|---|---|---|
| Salzsäure | 36% | 1,8 | 53% |
| Phosphorsäure | 84% | 3,4 | 52% |
| Bromwasserstoff | 48% | 3,0 | 48% |
| Trifluoressigsäure | 96% | 3,0 | 84%* |
| Ameisensäure | 98% | 2,4 | 77%* |
| Methansulfonsäure | 92% | 1,2 | 60% |

* in Lösung

## Patentansprüche

### Patentansprüche für folgende Vertragsstaaten : AT, BE, CH, DE, DK, FR, GB, IT, LI, LU, NL, SE

1. Verfahren zur Herstellung von Quadratsäure, dadurch gekennzeichnet, dass man einen Destillationsrückstand der Diketen-Produktion, der 3-Acetoxy-2-cyclobuten-1-on der Formel I enthält

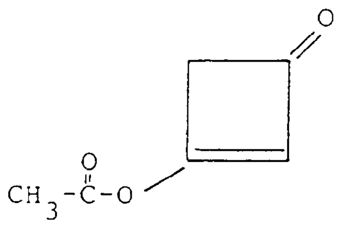

I

oder reines 3-Acetoxy-2-cyclobuten-1-on, entweder

a) in einer ersten Stufe durch Halogenierung zu einem Cyclobutenon der allgemeinen Formel

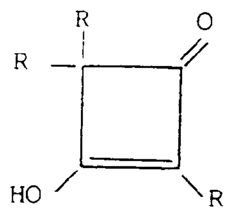

II

worin R gleichbedeutend ist und ein Chlor- oder Bromatom bedeutet, umsetzt, dieses Zwischenprodukt gegebenenfalls isoliert und dann in einer zweiten Stufe zur Quadratsäure der Formel

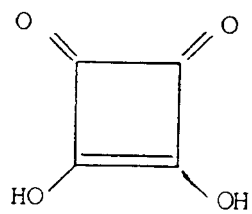

III

hydrolisiert oder

b) in einer ersten Stufe durch saure Hydrolyse in das 1,3-Cyclobutandion überführt, dieses Zwischenprodukt isoliert, dieses dann in einer zweiten Stufe durch Halogenierung zum Cyclobutenon der allgemeinen Formel II umsetzt, dieses Zwischenprodukt gegebenenfalls isoliert und dann in einer dritten Stufe zur Quadratsäure hydrolysiert.

2. Verfahren nach Patentanspruch 1, dadurch gekennzeichnet, dass man einen Destillationsrückstand der Diketen-Produktion mit einem Gehalt an 3-Acetoxy-2-cyclobuten-1-on von 5 bis 60 Gew.% als Ausgangsmaterial einsetzt.

3. Verfahren nach mindestens einem der Patentansprüche 1 bis 2, dadurch gekennzeichnet, dass man die Halogenierung von 3-Acetoxy-2-cyclobuten-1-on mit Brom oder Chlor durchführt.

4. Verfahren nach mindestens einem der Patentansprüche 1 bis 3, dadurch gekennzeichnet, dass man die Halogenierung von 3-Acetoxy-2-cyclobuten-1-on bei einer Temperatur von 10 bis 80°C durchführt.

5. Verfahren nach Patentanspruch 1, dadurch gekennzeichnet, dass man die saure Hydrolyse zum 1,3-Cyclobutandion mit wässriger Ameisensäure oder wässriger Schwefelsäure durchführt.

6. Verfahren nach mindestens einem der Patentansprüche 1 bis 5, dadurch gekennzeichnet, dass man die saure Hydrolyse bei einer Temperatur von 0 bis 30°C durchführt.

7. Verfahren nach mindestens einem der Patentansprüche 1 und 5 bis 6, dadurch gekennzeichnet, dass man die Halogenierung von 1,3-Cyclobutandion mit Brom oder Chlor durchführt.

8. Verfahren nach mindestens einem der Patentansprüche 1 und 5 bis 7, dadurch gekennzeichnet, dass man die Halogenierung von 1,3-Cyclobutandion bei einer Temperatur von 0 bis 50°C durchführt.

9. Verfahren nach mindestens einem der Patentansprüche 1 bis 8, dadurch gekennzeichnet, dass man die Hydrolyse zur Quadratsäure mit Mineralsäure, z.B. konzentrierter Schwefelsäure, mit Sulfonsäuren, mit Carbonsäuren oder mit Wasser durchführt.

10. Verfahren nach mindestens einem der Patentansprüche 1 bis 9, dadurch gekennzeichnet, dass man die Hydrolyse zur Quadratsäure bei einer Temperatur von 50 bis 150°C durchführt.

11. Cyclobutenone der allgemeinen Formel II

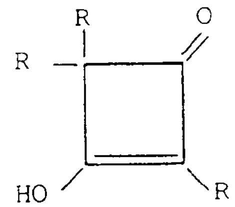

II

worin R gleichbedeutend ist und ein Chlor- oder Bromatom bedeutet, d.h., 2,4,4-Tribrom-3-hydroxy-2-cyclobuten-1-on oder 2,4,4-Trichlor-3-hydroxy-2-cyclobuten-1-on.

**Patentansprüche für folgenden Vertragsstaat : ES**

1. Verfahren zur Herstellung von Quadratsäure, dadurch gekennzeichnet, dass man einen Destillationsrückstand der Diketen-Produktion, der 3-Acetoxy-2-cyclobuten-1-on der Formel I enthält

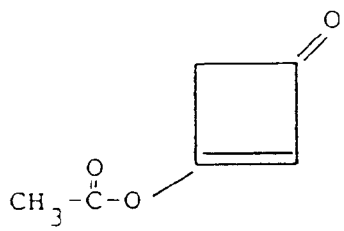

I

oder reines 3-Acetoxy-2-cyclobuten-1-on, entweder
a) in einer ersten Stufe durch Halogenierung zu einem Cyclobutenon der allgemeinen Formel

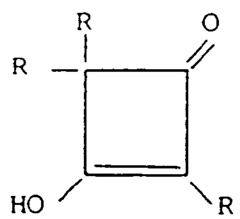

II

worin R gleichbedeutend ist und ein Chlor- oder Bromatom bedeutet, umsetzt, dieses Zwischenprodukt gegebenenfalls isoliert und dann in einer zweiten Stufe zur Quadratsäure der Formel

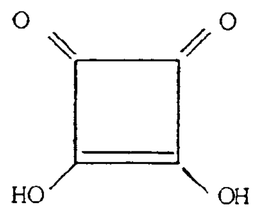

III

hydrolisiert oder

b) in einer ersten Stufe durch saure Hydrolyse in das 1,3-Cyclobutandion überführt, dieses Zwischenprodukt isoliert, dieses dann in einer zweiten Stufe durch Halogenierung zum Cyclobutenon der allgemeinen Formel II umsetzt, dieses Zwischenprodukt gegebenenfalls isoliert und dann in einer dritten Stufe zur Quadratsäure hydrolysiert.

2. Verfahren nach Patentanspruch 1, dadurch gekennzeichnet, dass man einen Destillationsrückstand der Diketen-Produktion mit einem Gehalt an 3-Acetoxy-2-cyclobuten-1-on von 5 bis 60 Gew.% als Ausgangsmaterial einsetzt.

3. Verfahren nach mindestens einem der Patentansprüche 1 bis 2, dadurch gekennzeichnet, dass man die Halogenierung von 3-Acetoxy-2-cyclobuten-1-on mit Brom oder Chlor durchführt.

4. Verfahren nach mindestens einem der Patentansprüche 1 bis 3, dadurch gekennzeichnet, dass man die Halogenierung von 3-Acetoxy-2-cyclobuten-1-on bei einer Temperatur von 10 bis 80°C durchführt.

5. Verfahren nach Patentanspruch 1, dadurch gekennzeichnet, dass man die saure Hydrolyse zum 1,3-Cyclobutandion mit wässriger Ameisensäure oder wässriger Schwefelsäure durchführt.

6. Verfahren nach mindestens einem der Patentansprüche 1 bis 5, dadurch gekennzeichnet, dass man die saure Hydrolyse bei einer Temperatur von 0 bis 30°C durchführt.

7. Verfahren nach mindestens einem der Patentansprüche 1 und 5 bis 6, dadurch gekennzeichnet, dass man die Halogenierung von 1,3-Cyclobutandion mit Brom oder Chlor durchführt.

8. Verfahren nach mindestens einem der Patentansprüche 1 und 5 bis 7, dadurch gekennzeichnet, dass man die Halogenierung von 1,3-Cyclobutandion bei einer Temperatur von 0 bis 50°C durchführt.

9. Verfahren nach mindestens einem der Patentansprüche 1 bis 8, dadurch gekennzeichnet, dass man die Hydrolyse zur Quadratsäure mit Mineralsäure, z.B. konzentrierter Schwefelsäure, mit Sulfonsäuren, mit Carbonsäuren oder mit Wasser durchführt.

10. Verfahren nach mindestens einem der Patentansprüche 1 bis 9, dadurch gekennzeichnet, dass man die Hydrolyse zur Quadratsäure bei einer Temperatur von 50 bis 150°C durchführt.

## Claims

### Claims for the following Contracting States : AT, BE, CH, DE, DK, FR, GB, IT, LI, LU, NL, SE

1. A process for the preparation of squaric acid, characterized in that a distillation residue of diketene production, which contains 3-acetoxy-2-cyclobuten-1-one of formula I:

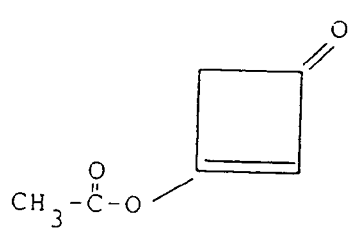

I

or pure 3-acetoxy-2-cyclobuten-1-one is either
(a) reacted in a first step by halogenation to a cyclobutenone of the general formula:

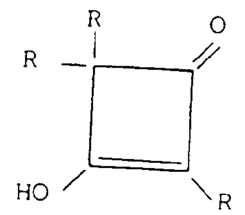

II

wherein each R has the same meaning and is chlorine or bromine; optionally followed by isolation of said intermediate product, and then hydrolized in a second step to squaric acid of the formula:

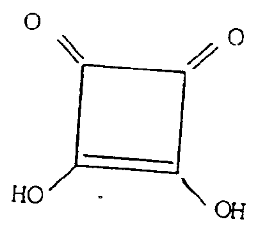

III

or:
(b) is converted in a first step by acid hydrolysis into the 1,3-cyclobutane dione, followed by isolation of said intermediate product which is then reacted in a second step by halogenation to the cyclobutenone of general formula II, whereupon this intermediate product is optionally isolated and then hydrolized in a third step to squaric acid.

2. The process according to Claim 1, characterized in that a distillation residue of diketene production having a content of 3-acetoxy-2-cyclobuten-1-one of 5 to 60% by weight is used as a starting material.

3. The process according to at least one of Claims 1 and 2, characterized in that halogenation of 3-acetoxy-2-cyclobuten-1-one is conducted with bromine or chlorine.

4. The process according to at least one of Claims 1 to 3, characterized in that halogenation of 3-acetoxy-2-cyclobuten-1-one is conducted at a temperature of from 10 to 80°C.

5. The process according to Claim 1, characterized in that acid hydrolysis to form 1,3-cyclobutane dione is conducted with aqueous formic acid or aqueous sulfuric acid.

6. The process according to at least one of Claims 1 to 5, characterized in that acid hydrolysis is conducted at a temperature of from 0 to 30°C.

7. The process according to at least one of Claims 1 and 5 to 6, characterized in that halogenation of 1,3-cyclobutane dione is conducted with bromine or chlorine.

8. The process according to at least one of Claims 1 and 5 to 7 characterized in that halogenation to 1,3-cyclobutane dione is conducted at a temperature of from 0 to 50°C.

9. The process according to at least one of Claims 1 to 8 characterized in that hydrolysis to squaric acid is conducted with mineral acids such as concentrated sulfuric acid, with sulfonic acids, with carboxylic acids, or with water.

10. The process according to at least one of Claims 1 to 9 characterized in that hydrolysis to squaric acid is conducted at a temperature of from 50 to 150°C.

11. Cyclobutenone of the general formula II:

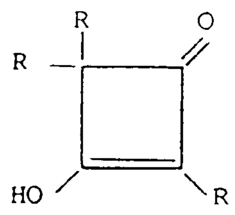

II

wherein each R has the same meaning and is chlorine or bromine; i.e. 2,4,4-tribromo-3-hydroxy-2-cyclobuten-1-one, or 2,4,4-trichloro-3-hydroxy-2-cyclobuten-1-one.

**Claims for the following Contracting State : ES**

1. A process for the preparation of squaric acid, characterized in that a distillation residue of diketene production, which contains 3-acetoxy-2-cyclobuten-1-one of formula I:

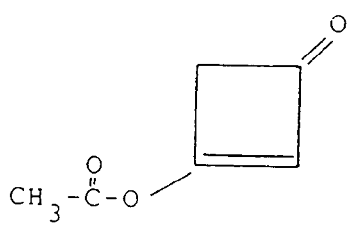

I

or pure 3-acetoxy-2-cyclobuten-1-one is either
(a) reacted in a first step by halogenation to a cyclobutenone of the general formula:

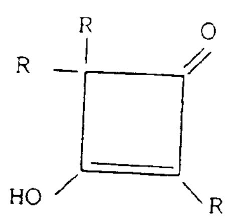

II

wherein each R has the same meaning and is chlorine or bromine; optionally followed by isolation of said intermediate product, and then hydrolized in a second step to squaric acid of the formula:

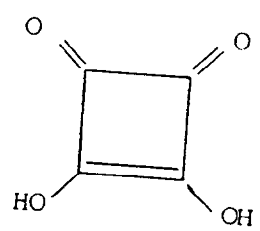

III

or:
(b) is converted in a first step by acid hydrolysis into the 1,3-cyclobutane dione, followed by isolation of said intermediate product which is then reacted in a second step by halogenation to the cyclobutenone of general formula II, whereupon this intermediate product is optionally isolated and then hydrolized in a third step to squaric acid.

2. The process according to Claim 1, characterized in that a distillation residue of diketene production having a content of 3-acetoxy-2-cyclobuten-1-one of 5 to 60% by weight is used as a starting material.

3. The process according to at least one of Claims 1 and 2, characterized in that halogenation of 3-acetoxy-2-cyclobuten-1-one is conducted with bromine or chlorine.

4. The process according to at least one of Claims 1 to 3, characterized in that halogenation of 3-acetoxy-2-cyclobuten-1-one is conducted at a temperature of from 10 to 80°C.

5. The process according to Claim 1, characterized in that acid hydrolysis to form 1,3-cyclobutane dione is conducted with aqueous formic acid or aqueous sulfuric acid.

6. The process according to at least one of Claims 1 to 5, characterized in that acid hydrolysis is conducted at a temperature of from 0 to 30°C.

7. The process according to at least one of Claims 1 and 5 to 6, characterized in that halogenation of 1,3-cyclobutane dione is conducted with bromine or chlorine.

8. The process according to at least one of Claims 1 and 5 to 7 characterized in that halogenation of 1,3-cyclobutane dione is conducted at a temperature of from 0 to 50°C.

9. The process according to at least one of Claims 1 to 8 characterized in that hydrolysis to squaric acid is conducted with mineral acids such as concentrated sulfuric acid, with sulfonic acids, with carboxylic acids, or with water.

10. The process according to at least one of Claims 1 to 9 characterized in that hydrolysis to squaric acid is conducted at a temperature of from 50 to 150°C.

## Revendications

**Revendications pour les Etats contractants suivants : AT, BE, CH, DE, DK, FR, GB, IT, LI, LU, NL, SE**

1. Procédé pour la préparation de l'acide carré, caractérisé en ce que l'on fait réagir un résidu de distillation de la production de dicétène, qui contient la 3-acétoxy-2-cyclobuten-1-one de la formule I

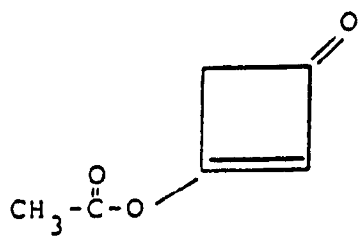

I

ou une 3-acétoxy-2-cyclobuten-1-one pure, soit

a) dans une première étape par halogénation en une cyclobuténone de la formule générale

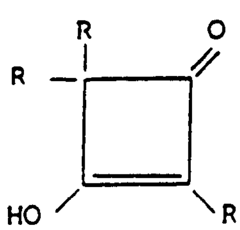

II

dans laquelle R est identique et signifie un atome de chlore ou de brome, ce produit intermédiaire étant éventuellement isolé puis dans une deuxième étape hydrolysé en acide carré

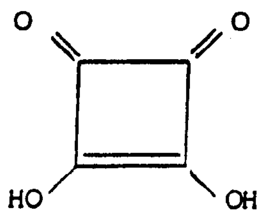

III

ou

b) dans une première étape par hydrolyse acide transformé en 1,3-cyclobutandione, ce produit intermédiaire étant isolé puis dans une seconde étape par halogénation transformé en cyclobuténone de la formule générale II, ce produit intermédiaire étant éventuellement isolé puis dans une troisième étape hydrolysé en acide carré.

2. Procédé selon la revendication 1, caractérisé en ce que l'on met en oeuvre un résidu de distillation de la production de dicétène avec une teneur en 3-acétoxy-2-cyclobuten-1-one de 5 à 60 % en poids en tant que matériau de départ.

3. Procédé selon au moins l'une des revendications 1 à 2, caractérisé en ce que l'on effectue l'halogénation de 3-acétoxy-2-cyclobuten-1-one avec du brome ou du chlore.

4. Procédé selon au moins l'une des revendications 1 à 3, caractérisé en ce que l'on effectue l'halogénation de 3-acétoxy-2-cyclobuten-1-one à une température de 10 à 80°C.

5. Procédé selon la revendication 1, caractérisé en ce que l'on effectue l'hydrolyse acide en 1,3-cyclobutandione avec de l'acide formique aqueux ou de l'acide sulfurique aqueux.

6. Procédé selon au moins l'une des revendications 1 à 5, caractérisé en ce que l'on effectue l'hydrolyse acide à une température de 0 à 30°C.

7. Procédé selon au moins l'une des revendications 1 et 5 à 6, caractérisé en ce que l'on effectue l'halogénation de 1,3-cyclobutandione avec du brome ou du chlore.

8. Procédé selon au moins l'une des revendications 1 et 5 à 7, caractérisé en ce que l'on effectue l'halogénation du 1,3-cyclobutandione à une température de 0 à 50°C.

9. Procédé selon au moins l'une des revendications 1 à 8, caractérisé en ce que l'on effectue l'hydrolyse en acide carré avec de l'acide minéral par exemple de l'acide sulfurique concentré avec des acides sulfoniques, avec des acides carboxyliques ou avec de l'eau.

10. Procédé selon au moins l'une des revendications 1 à 9, caractérisé en ce que l'on effectue l'hydrolyse en acide carré à une température de 50 à 150°C.

11. Cyclobuténones de la formule générale II

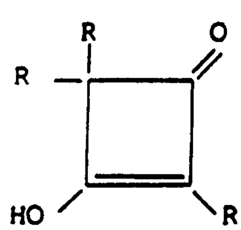

II

dans laquelle R est identique et signifie un atome de chlore ou de brome, c'est-à-dire la 2,4,4-tribrom-3-hydroxy-2-cyclobuten-1-one ou la 2,4,4-trichlor-3-hydroxy-2-cyclobuten-1-one.

**Revendications pour l'Etat contractant suivant : ES**

1. Procédé pour la préparation d'acide carré, caractérisé en ce que l'on met en réaction un résidu de distillation de la production de dicétène, qui contient la 3-acétoxy-2-cyclobuten-1-one de la formule I

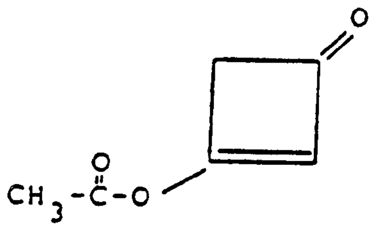

I

ou une 3-acétoxy-2-cyclobuten-1-one pure, soit

a) dans une première étape par halogénation en une cyclobuténone de la formule générale

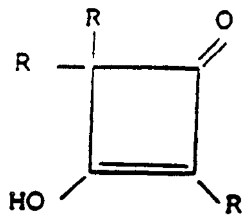

II

dans laquelle R est identique et signifie un atome de chlore ou de brome, ce produit intermédiaire étant éventuellement isolé puis dans une deuxième étape hydrolysé en acide carré

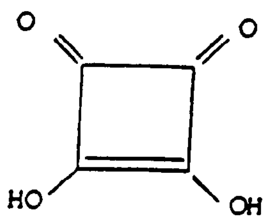

III

ou

b) dans une première étape par hydrolyse acide transformé en 1,3-cyclobutandione, ce produit intermédiaire étant isolé puis dans une seconde étape par halogénation transformé en cyclobuténone de la formule générale II, ce produit intermédiaire étant éventuellement isolé puis dans une troisième étape hydrolysé en acide carré.

2. Procédé selon la revendication 1, caractérisé en ce que l'on met en oeuvre un résidu de distillation de la production de dicétène avec une teneur en 3-acétoxy-2-cyclobuten-1-one de 5 à 60 % en poids en tant que matériau de départ.

3. Procédé selon au moins l'une des revendications 1 à 2, caractérisé en ce que l'on effectue l'halogénation de 3-acétoxy-2-cyclobuten-1-one avec du brome ou du chlore.

4. Procédé selon au moins l'une des revendications 1 à 3, caractérisé en ce que l'on effectue l'halogénation de 3-acétoxy-2-cyclobuten-1-one à une température de 10 à 80°C.

5. Procédé selon la revendication 1, caractérisé en ce que l'on effectue l'hydrolyse acide en 1,3-cyclobutandione avec de l'acide formique aqueux ou de l'acide sulfurique aqueux.

6. Procédé selon au moins l'une des revendications 1 à 5, caractérisé en ce que l'on effectue l'hydrolyse acide à une température de 0 à 30°C.

7. Procédé selon au moins l'une des revendications 1 et 5 à 6, caractérisé en ce que l'on effectue l'halogénation de 1,3-cyclobutandione avec du brome ou du chlore.

8. Procédé selon au moins l'une des revendications 1 et 5 à 7, caractérisé en ce que l'on effectue l'halogénation du 1,3-cyclobutandione à une température de 0 à 50°C.

9. Procédé selon au moins l'une des revendications 1 à 8, caractérisé en ce que l'on effectue l'hydrolyse en acide carré avec de l'acide minéral par exemple de l'acide sulfurique concentré avec des acides sulfoniques, avec des acides carboxyliques ou avec de l'eau.

10. Procédé selon au moins l'une des revendications 1 à 9, caractérisé en ce que l'on effectue l'hydrolyse en acide carré à une température de 50 à 150°C.